Europäisches Patentamt

European Patent Office

Office européen des brevets

(11) Publication number: **0 056 740**
**A1**

(12) **EUROPEAN PATENT APPLICATION**

(21) Application number: 82300318.1

(22) Date of filing: 21.01.82

(51) Int. Cl.³: **C 07 D 301/19**
**B 01 J 23/92, C 22 B 34/34**

(30) Priority: 21.01.81 US 227114

(43) Date of publication of application:
28.07.82 Bulletin 82/30

(84) Designated Contracting States:
BE DE FR GB IT NL

(71) Applicant: ATLANTIC RICHFIELD COMPANY
Arco Plaza 515 S. Flower Street
Los Angeles California 90071(US)

(72) Inventor: Mocella, Michael Thomas
308 Baywood Road
West Chester Pennsylvania 19380(US)

(74) Representative: Cropp, John Anthony David et al,
MATHYS & SQUIRE 10 Fleet Street
London, EC4Y 1AY(GB)

(54) Production of a solid molybdenum precipitate from a spent molybdenum epoxidation catalyst solution.

(57) In the process of recovering molybdenum from a spent catalyst solution which is obtained from a molybdenum catalyzed epoxidation of an olefin with an organic hydroperoxide by removing from the crude epoxidation reaction mixture product epoxide and the alcohol corresponding to the hydroperoxide, said process comprises removing and recovering dissolved molybdenum as a molybdenum containing solid by mixing the spent catalyst solution with water in an amount sufficient to produce a two phase system comprising an organic phase and an aqueous phase, heating the resulting aqueous phase which is rich in molybdenum values to precipitate a molybdenum-containing solid and separating the solid.

EP 0 056 740 A1

PRODUCTION OF A SOLID MOLYBDENUM PRECIPITATE

FROM A SPENT MOLYBDENUM EPOXIDATION CATALYST SOLUTION


Oxirane compounds such as ethylene oxide, propylene oxide, and their higher homologs are valuable articles of commerce.  One of the most attractive processes for synthesis of those oxirane compounds is described by Kollar in United States Patent No. 3,351,635.  According to Kollar, the oxirane compound (e.g., propylene oxide) may be prepared by epoxidation of an olefinically unsaturated compound (e.g., propylene) by use of an organic hydroperoxide and a suitable catalyst such as molybdenum.

During the epoxidation reaction the hydroperoxide is converted almost quantitatively to the corresponding alcohol. That alcohol may be recovered as a coproduct with the oxirane compound.  However, it is the oxirane which is of primary concern.

Kollar teaches that oxirane compounds may be prepared from a wide variety of olefins.  Lower olefins having three or four carbon atoms in an aliphatic chain are advantageously epoxidized by the process.  The class of olefins commonly termed alpha olefins or primary olefins are epoxidized in a particularly efficient manner by the process.  It is known to those in the art that primary olefins, e.g., propylene, butene-1, decene-1 hexadecene-1 etc., are much more difficulty expoxidized than other forms of olefins, excluding only ethylene. Other forms of
olefins which are much more easily epoxidized are substituted
olefins, alkenes with internal unsaturation, cycloalkenes and the
like. Kollar teaches that notwithstanding the relative
difficulty in epoxidizing primary olefins, epoxidation proceeds
more efficiently when molybdenum, titanium or tungsten catalysts
are used. Molybdenum is of special interest. Kollar teaches
that activity of those metals for epoxidation of the primary
olefins is surprisingly high and can lead to high selectivity of
propylene to propylene oxide. These high selectivities are
obtained at high conversions of hydroperoxide (50% or higher)
which conversion levels are important for commercial utilization
of the technology.

Kollar's epoxidation reaction proceeds under pressure
in the liquid state and, accordingly, a liquid solution of the
metal catalyst is preferred. Preparation of a suitable catalyst
is taught by Sheng et al in United States Patent No. 3,434,975.
According to Sheng, the reaction-medium soluble epoxidation
catalyst may be prepared by reacting molybdenum metal with an
organic hydroperoxide, per acid or hydrogen peroxide in the
presence of a saturated alcohol having one to four carbon atoms.

When propylene is epoxidized with tertiary-butyl
hydroperoxide according to the Kollar process using the Sheng
catalyst, a product mixture containing unreacted propylene,
propylene oxide, tertiary-butyl alcohol and molybdenum catalyst is obtained. Distillation of that product mixture provides substantially pure propylene oxide and tertiary-butyl alcohol. The residue of distillation (hereafter "TBA bottoms") contains spent molybdenum catalyst as well as high boiling organic residues.

Removal and recovery of the molybdenum values from the distillation residue are important from ecological and economical standpoints. In United States Patent No. 3,763,303 Khori et al disclose two embodiments of a process for recovering molybdenum values from spent epoxidation catalysts. The Khori process first embodiment involves recovery of molybdenum directly from the spent catalyst mixture by a liquid-to-liquid extraction utilizing an aqueous extractant consisting essentially of water which is intermittently admixed with the residue to be treated to effect an extraction and transfer of a portion of the molybdenum constituent from the organic phase to the aqueous phase. Untreated spent catalyst solutions usually contain molybdenum in concentrations of from about 0.1% to about 1.0% by weight and Khori discloses those solutions are highly satisfactory for treatment in the liquid-to-liquid extraction process in which the extractant consists essentially of water to effect molybdenum separation. Molybdenum separated with the aqueous extract is recovered as molybdenum trioxide by evaporation of water followed by calcination of the solid obtained by extract evaporation.

The second embodiment of the Khori process relates to extracting molybdenum from distillation residues obtained from distillation of spent catalyst solution (TBA bottoms) but the extraction is performed with acids or bases to convert the molybdenum into a recoverable molybdenum compound of the acid or base.

British Patent Specification 1,317,480 also teaches recovery of molybdenum values from spent epoxidation catalysts. As in Khori, the British recovery process involves extracting the spent catalyst solution with water alone or with aqueous ammonia. The British extraction process results in a transfer of at least 95% of the available molybdenum values to the aqueous extract. Those molybdenum values are recovered from the aqueous phase by precipitation as a phosphomolybdate or by distillative stripping of the volatile organic material and water from the extract.

The spent catalyst solution may also be subjected to exhaustive evaporation or distillation to produce a residue with a higher molybdenum content as taught by Levine et al in United States Patent No. 3,819,663. The Levine process starts with a spent catalyst solution such as TBA bottoms and subjects that solution to a wiped film evaporation at 375 to 450°F until 60 to 80% by weight of the solution is evaporated overhead. The residue of that evaporation is taught to be useful as a catalyst in further epoxidation processes.

Lemke teaches in United States Patent No. 3,887,361 that molybdenum may be precipitated from spent catalyst solutions such as TBA bottoms. To effect precipitation Lemke discloses adding tertiary-butyl alcohol to the TBA bottoms until a level of 5 to 50% by weight of tertiary-butyl alcohol is achieved. Then the mixture is heated to 100 to 300°C to effect molybdenum precipitation. The precipitated molybdenum can then be reused in further epoxidations.

The present invention provides a means for recovering soluble molybdenum from a spent catalyst solution produced in the molybdenum catalyzed epoxidation of an olefin with a hydroperoxide. The molybdenum is recovered from the spent catalyst solution as a solid material which is produced by adding sufficient water to the spent catalyst solution with agitation, the amount of water being sufficient to cause separation into an aqueous phase and an organic phase. Heating the resulting aqueous molybdenum-rich solution produces a solid molydenum compound which may be separed.

As used in the present specification and the annexed claims, the term "spent catalyst solution" is intended to mean that fraction of the epoxidation reaction product effluent remaining after removal of unreacted olefin (for example, propylene), alkylene oxide (for example, propylene oxide) and a major portion of the alcohol corresponding to the hydroperoxide (for example, tertiary butyl hydroperoxide) used in the epoxidation reaction which reaction may be according to the procedure of Kollar, the teachings of which are hereby incorporated by reference in their entirity. Spent catalyst solution, apart from molybdenum compounds, contains some alcohol, acids and other low molecular weight oxygenated compounds and said spent catalyst solution is generally not subjected to any chemical treatment before being subjected to the process of the present invention. It is contemplated that spent catalyst solution as used herein includes both the distillation bottoms treated in British Patent Specification 1,317,480 and the residue obtained from the wiped film evaporation process according to Levine and said spent catalyst solution can contain molybdenum compounds at levels of up to 5% by weight.

In one aspect of the present invention the process of separating the components of a reaction mixture obtained from epoxidizing an olefin with an organic hydroperoxide in the presence of a liquid molybdenum catalyst wherein the product epoxide and the alcohol corresponding to the hydroperoxide are removed from the reaction mixture leaving a spent catalyst solution is improved by removing and recovering dissolved molybdenum as a molybdenum containing solid by mixing the spent catalyst solution with sufficient water to produce a two phase system comprising an aqueous phase solution rich in molybdenum and an organic phase of undissolved spent catalyst solution, heating the aqueous molybdenum-rich solution to precip itate a molybdenum containing solid and separating the solid.

Another aspect of the present invention relates to the process of recovering molybdenum from a spent catalyst solution which is obtained from a molybdenum catalyzed epoxidation of an olefin with an organic hydroperoxide by removing from the crude epoxidation reaction mixture product epoxide and the alcohol corresponding to the hydroperoxide, said process comprises removing and recovering dissolved molybdenum as a molybdenum containing solid by mixing the spent catalyst solution with sufficient water to produce a two phase system comprising an aqueous phase molybdenum-rich solution and an organic phase of undissolved spent catalyst solution, heating the aqueous molybdenum-rich solution to precipitate a molybdenum-containing solid and separating the solid.

Accordingly, it is an object of the present invention to provide a simple and speedy process for efficiently recovering molybdenum from spent catalyst solutions in the form of a solid compound which is directly suitable for use in preparation of fresh soluble molybdenum epoxidation catalyst.

Another object of this invention is to provide a more efficient and economical method for removal of dissolved molybdenum catalysts from spent epoxidation catalyst solutions. By the present process the transfer of molybdenum values into the aqueous phase is so large that the remaining organic phase residue may contain less than about 250, and preferably, less than 100 parts per million of molybdenum.

Still another object of the invention is to provide a process wherein the organic part of the spent catalyst solution is substantially freed of molybdenum, rendering the organic portion suitable for use or sale as a fuel.

These and other objects of the invention will become apparent from the following description of the invention and the examples.

The present invention involves a simple and speedy yet highly effective means for separating organic residues from molybdenum values in spent catalyst solutions from hydroperoxide oxidations of olefins. By use of the Present invention, the level of molybdenum contained in the organic residues is reduced to a very low level which permits disposal of the organic residue by sale for fuel value, use as a fuel or by other conventional means without equipment fouling or pollution of the environment. In the present process, transfer of molybdenum into the aqueous phase leaves an organic residue substantially free of molybdenum. Transfer of substantially all the molybdenum values into the aqueous phase is important for both molybdenum recovery from the aqueous phase as well as for purification of the organic phase residue. The organic phase residue is itself an important commodity which can be sold as a fuel or burned directly as a fuel. However, in order to be useful for fuel purposes, the molybdenum level should be lower than about 250, and preferably, lower than 100 parts per million molybdenum in the organic phase residue.  Higher levels of molybdenum in the organic phase residue burned as fuel results in fouling of the furnace burning the fuel.  As such frequent furnace downtime is necessitated for cleaning and repair.  Also, levels of molybdenum higher than about 250 ppm, may result in objectionable increases in  environ-mental  pollution.

Furthermore, this invention provides a means for recovering solid molybdenum compounds which are _per se_ valuable for their molydenum content and which may be sold for the molybdenum content or used without further processing as the source of molybdenum used in preparation of a soluble molybdenum epoxidation catalyst according to the procedures known in the art or in the procedure of my copending application entitled "Preparation of Soluble Molybdenum Catalysts for Epoxidation of Olefins" (internal docket no. 80-PD-13/80-PD-20) filed concurrently herewith.

The aqueous solution also contains some dissolved organic material from the spent catalyst solution.  Those organic materials may also be separated from the aqueous solution prior to disposal of the purified aqueous phase.

The molybdenum catalyzed hydroperoxide oxidation (epoxidation) of olefins from which the spent catalyst solution to be treated is obtained is described by Kollar, discussed above.  Kollar describes a wide variety of olefins which can be epoxidized by a wide variety of peroxides under the influence of molybdenum catalysts. The present invention is applicable to separating solid molybdenum compounds from the organic residues of spent catalyst solutions obtained from the epoxidation of any of the olefins disclosed by Kollar using any of the peroxides disclosed by Kollar. Thus the present invention has general applicability for separating molybdenum from the organic residue of a spent epoxidation catalyst stream.

Epoxidation of an olefin is suitably accomplished by charging a reactor with the olefin to be oxidized, an organic hydroperoxide and a soluble molybdenum catalyst prepared according to the art. After epoxidation under the conditions taught by Kollar, product epoxide is removed from the reaction mixture leaving a by-product mixture containing, inter alia, spent catalyst solution and an alcohol corresponding to the hydroperoxide reactant employed. For instance, when tertiary-butyl hydroperoxide is employed in the epoxidation, tertiary-butyl alcohol is formed. The alcohol is removed from the by-product mixture by distillation which leaves as a residue the spent catalyst solution containing molydenum values and higher molecular weight organic residues.

To recover molybdenum from the spent catalyst solution, water is added to the spent catalyst solution with agitation. A sufficient quantity of water to cause formation of an aqueous phase and a distinct organic phase is necessary for this process. A weight ratio of water to spent catalyst solution of from 1:4 to 4:1 is sufficient. However, the best molybdenum recovery is unexpectedly observed when about equal weights of water and spent catalyst solution are mixed.

After the spent catalyst solution has been thoroughly mixed with water, molybdenum precipitation is achieved by heating the mixture to a temperature of 120 to 225°C. Preferred temperatures are above 150°C with the most preferred being about 200°C. Still higher temperatures produce faster precipitation but heating at such temperatures may be accompanied by undesirable side effects, such as increased corrosion rates. The heating time required for substantially complete precipitation of a solid molybdenum compound is from 15 minutes to 3 hours and to a considerable extent is dependent on the precipitation temperature employed with higher temperatures requiring shorter periods of time for precipitation. Sufficient temperatures to effect precipitation within 15 to 60 minutes are preferred.

Heating the two phase mixture of water and spent catalyst solution together at the necessary precipitation temperature results in precipitation of high levels of solid molybdenum compounds and leaves advantageously low levels (to less than 100 ppm) of residual dissolved molybdenum in either the remaining aqueous phase or the organic phase. While it is contemplated that the simplest method of precipitation of molybdenum compound is from a heated admixture of aqueous and organic phases, it is within the scope of this invention to separate the aqueous phase from the organic phase prior to heating the aqueous phase alone to effect molybdenum
precipitation therefrom.   The process disclosed and claimed in
copending application entitled "Recovery of Molybdenum as an
Aqueous Solution" (internal docket no. 80-PD-16) filed
concurrently herewith provides a suitable procedure for producing
and separating an aqueous phase rich in molybdenum from spent
catalyst solutions.

Precipitated solids may be separated from the remaining
liquid by any conventional solid/liquid separating means.   For
example, precipitated solids may be isolated by filtration,
centrifugation or merely sedimentation followed by decanting the
supernatant liquid.   The liquid from which the solid is removed
may be eliminated in any suitable fashion without undue
molybdenum pollution.

The solids obtained in the present invention have not
been fully characterized and the chemical structure is not
presently known.   However, the solids differ from other
molybdenum containing solids which have been recovered by prior
art processes such as, for example, the Lemke process discussed
above.   The solids of this invention have been studied by
infrared spectroscopy and pyrolysis gas chromatograph mass
spectroscopy.   Unfortunately, translation of the data obtained to
ascertain molecular structures is not straight forward.   It is
believed, however, that the solids obtained are organo-
molybdenum complexes with the molybdenum being largely in the +6 oxidation state. Some physical characteristics of the solid precipitate include:

    I. A typical elemental analysis by weight percent is 6% carbon, 1.2% hydrogen, 28.7% oxygen and the balance molybdenum.

    II. Prominent infrared spectrum bands at 970 cm.$^{-1}$ and 750 cm.$^{-1}$ are consistent with Mo = 0 and Mo - 0 - Mo moieties.

    III. A sample of the solid was pyrolyzed by very rapid heating to 600$^{\circ}$C in a stream of helium and the pyrolysis products analyzed. Besides routine CO, $CO_2$ and $H_2O$, the materials in significant quantities from the pyrolysis were acetaldehyde and acetone.

    In order to further illustrate the subject matter of the present invention, the following examples are provided. However, it is to be understood that the examples are merely illustrative and are not intended as being restrictive of the invention herein disclosed and as defined by the annexed claims.

## EXAMPLE 1

Equal weights of distilled water and spent catalyst solution, more fully described below, (20 grams each) were charged into a 125cc pressure reactor. The reactor was sealed, pressurized to 100 psi with nitrogen and immersed in a thermostatically controlled oil bath. After a 6-8 minute heat-up period, the reactor was heated at 175°C for 15 minutes. After the reaction time, the reactor was cooled and vented. The reaction mixture was then centrifuged to separate the precipitated solid from the aqueous and organic liquid phases. Based on analysis of soluble molybdenum remaining in the liquid phases, it was calculated that 79.7% of the original molybdenum had been removed as precipitated solid.

The spent catalyst solution treated according to this example originally contained about 0.4% dissolved molybdenum by weight. The spent catalyst solution was obtained as the distillation bottoms resulting from distillation of propylene oxide and tertiary-butyl alcohol from a crude reaction product obtained from the molybdenum catalyzed epoxidation of propylene with tertiary-butyl hydroperoxide.

- 15 -

EXAMPLES 2-12

The procedure of Example 1 was repeated for each of Examples 2-12 using the varying conditions indicated below.

| Example | Grams Charged | | Reaction Conditions | | |
| --- | --- | --- | --- | --- | --- |
| | Spent Catalyst Solution | $H_2O$ | T ($^{o}$C) | Time (min.) | Percent Mo Precipitated |
| 2 | 20 | 20 | 175 | 30 | 79.8 |
| 3 | 20 | 20 | 175 | 45 | 89.9 |
| 4 | 20 | 20 | 175 | 60 | 91.2 |
| 5 | 20 | 20 | 200 | 15 | > 99.1 |
| 6 | 20 | 20 | 200 | 30 | > 99.2 |
| 7 | 20 | 20 | 200 | 45 | > 99.2 |
| 8 | 20 | 20 | 200 | 60 | > 99.3 |
| 9 | 32 | 8 | 200 | 30 | 93.7 |
| 10 | 26 | 13 | 200 | 30 | 90.8 |
| 11 | 13 | 26 | 200 | 30 | 98.8 |
| 12 | 8 | 32 | 200 | 30 | 97.5 |

These Examples clearly show that the extent of preciptiation increases with both heating time and temperature and that at constant heating times and temperatures, precipitation reaches a maximum when about equal weights of water and spent catalyst solution are used.

EXAMPLE 13

This example illustrates the embodiment of this invention wherein the spent catalyst solution was extracted with water and the aqueous extract alone heated to precipitate a solid molybdenum containing compound.

Extraction of an equal weight of spent catalyst solution with water at room temperature for one hour resulted in a 60% transfer of total molybdenum content to the aqueous phase. A similar extraction at $100^{\circ}C$ for one hour resulted in greater than 90% transfer of total molybdenum to the aqeuous phase. Heating to $175^{\circ}C$ caused precipitation to proceed efficiently. When the aqueous extract was heated at $200^{\circ}C$ for 15 minutes, the level of soluble molybdenum was reduced to below 100 ppm.

EXAMPLE 14

Equal weights of distilled water and spent catalyst solution containing 4270 ppm soluble molybdenum were stirred together for two hours at room temperature. Separation of the two resulting liquid phases yielded an organic raffinate with 1260 ppm soluble molybdenum and a deep blue aqueous extract containing 2290 ppm soluble molybdenum. The aqueous extract was heated at $200^{\circ}C$ in a closed vessel for one hour which resulted in precipitation of 99% of the soluble molybdenum as a black powder. The remaining pale yellow aqueous liquid contained only 20 ppm soluble molybdenum.

## EXAMPLE 15

Distilled water (50 grams) and a spent catalyst solution (50 grams) containing 4270 ppm soluble molybdenum were stirred together for one hour at a temperature of $20^{\circ}$C. The mixture was then permitted to stand overnight to effect phase separation. The organic raffinate was removed and was found to contain 2020 ppm soluble molybdenum. The aqueous extract contained 2080 ppm dissolved molybdenum. The aqueous extract was then heated to $175^{\circ}$C for a period of 60 minutes. Thereupon about 57% of the original dissolved molybdenum was precipitated as a solid and the remaining aqueous liquid contained very little soluble molybdenum.

## EXAMPLE 16

Distilled water (50 grams) and a spent catalyst solution (50 grams) containing 4270 ppm soluble molybdenum were stirred together for 18 hours at a temperature of $100^{\circ}$C. The mixture was then permitted to stand overnight to effect phase separation. The organic raffinate was removed and was found to contain 197 ppm soluble molybdenum. The aqueous extract contained 3080 ppm dissolved molybdenum. The aqueous extract was then heated to $175^{\circ}$C for a period of 30 minutes. Thereupon about 84% of the original dissolved molybdenum was precipitated as a solid and the remaining aqueous liquid contained 450 ppm soluble molybdenum.

— 1 —

CLAIMS

1.  A process of separating the components of a reaction mixture obtained from epoxidizing an olefin with an organic hydroperoxide in the presence of a liquid molybdenum catalyst wherein the product epoxide and the alcohol corresponding to the hydroperoxide are removed from the reaction mixture leaving a spent catalyst solution, characterised in that dissolved molybdenum is removed and recovered as a molybdenum containing solid by mixing the spent catalyst solution with water in an amount sufficient to produce a two phase system comprising an organic phase and an aqueous phase, heating the resulting aqueous phase which is rich in molybdenum values to precipitate a molybdenum-containing solid and separating the solid.

2.  A process of recovering molybdenum from a spent catalyst solution which is obtained from a molybdenum catalyzed epoxidation of an olefin with an organic hydroperoxide by removing from the crude epoxidation reaction mixture product epoxide and the alcohol corresponding to the hydroperoxide, characterised in that said process comprises removing and recovering dissolved molybdenum as a molybdenum containing solid by mixing the spent catalyst solution with water in an amount sufficient to produce a two phase system comprising an organic phase and an aqueous phase, heating the resulting aqueous phase which is rich in molybdenum values to precipitate a molybdenum-containing solid and separating the solid.

3.  The process according to claim 1 or claim 2 wherein the aqueous phase is heated in the presence of the organic phase.

4.  The process according to claim 1 or claim 2 wherein the aqueous phase is separated from the organic phase prior to heating the aqueous solution.

5.  The process according to any one of claims 1 to 4 wherein the aqueous phase is heated at $120^{\circ}$ to $225^{\circ}C$ for 15 minutes to 3 hours.

6.  The process according to any one of claims 1 to 4 wherein the aqueous phase is heated at about $200^{\circ}C$ until precipitation of molybdenum is effected.

7.    The process according to any one of claims 1 to 6 wherein the ratio of water to spent catalyst solution is 1:4 to 4:1 parts by weight.

8.    The process according to any one of claims 1 to 7 wherein about equal parts by weight of water and spent catalyst solution are mixed.

9.    The molybdenum containing solid precipitated according to the process of any one of claims 1 to 8.

## European Patent Office

## EUROPEAN SEARCH REPORT

| Category | Citation of document with indication, where appropriate, of relevant passages | Relevant to claim |
|---|---|---|
| **DOCUMENTS CONSIDERED TO BE RELEVANT** | | |
| D,X | US - A - 3 763 303 (A. KHURI)  * the whole document, particularly column 8, lines 22-26 * | 1-9 |
| D,A | GB - A - 1 317 480 (ATLANTIC RICHFIELD)  * the whole document * | 1-9 |

**CLASSIFICATION OF THE APPLICATION (Int. Cl. 3)**

C 07 D 301/19
B 01 J 23/92
C 22 B 34/3 4

**TECHNICAL FIELDS SEARCHED (Int.Cl. 3)**

C 07 D 301/00
B 01 J 23/00
C 22 B 34/00
C 22 B 7/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 10-03-1982 | ALLARD |

EPO Form 1503.1   06.78